# EUROPEAN PATENT APPLICATION

(11) **EP 1 310 800 A2**
(43) Date of publication of application: **14.05.2003**
(21) Application number: 02257707.6
(22) Date of filing: 06.11.2002
(51) Int. Cl.: G01N 33/94, G01N 33/68

(54) **Functional assay for agonist activation of neuroreceptors**

(30) Priority: 09.11.2001 US 344755 P
(71) Applicant: Pfizer Products Inc., Groton, Connecticut 06340 (US)
(72) Inventor: Shrikhande, Alka Vinay, c/o Pfizer Global R & D, Groton, Connecticut 06340 (US); Wong, Stephen Kwok-Fung,c/o Pfizer Global R & D, Groton, Connecticut 06340 (US)
(74) Representative: England, Peter Michael

(57) **Abstract**

The invention provides a novel high throughput functional assay for certain agonist-activated receptors, including Alpha 1A , Alpha 2A, H1, 5HT1A, 5HT2A, D2 and D3 receptors. The assay method of the invention uses an elevated temperature and a cell line that stably expresses both the receptor and the promiscuous G protein Gα15 wherein agonist-induced intracellular Ca²⁺ release was monitored by a Fluorometric Imaging Plate Reader (FLIPR). The magnitude of the agonist-induced response was dramatically enhanced by performing the assay at an elevated temperature, rather than at room temperature. The novel assay of the invention is useful for selecting compounds which are effective in the treatment of disorders related to the activation of certain neuroreceptors.

## Description

### Background of the Invention

The present invention relates to a novel high throughput functional assay for certain agonist-activated receptors.

The novel assay of the present invention is useful for selecting compounds which are effective in the treatment of a wide variety of disorders related to the activation of certain neuroreceptors, including Alpha 2A, H1, 5HT1A, 5HT2A, D2 and D3 receptors. Molecules which modulate the functions of such receptors are potentially therapeutic towards various psychiatric diseases. While ligand binding assays have been used for the discovery of such molecules, assays to determine their functionality as receptor ligands are often more informative. Development of high throughput functional assays for activation of these receptors has been problematic.

The assay method of the invention uses elevated temperature in conjunction with a Fluorometric Imaging Plate Reader (FLIPR³⁸⁴) for measurement of agonist activation of such receptors. For example, the present invention relates to an improved assay method for the D3 dopamine receptor, a G protein-coupled receptor (GPCR) which activates the Gᵢ/Gₒ subtypes of G-proteins, and which is preferentially expressed in the limbic regions such as the septal area and amygdala, and is thought to be important for the regulation of cognition, motivation and emotion. The D3 receptor, like other receptors which may be assayed by the method of the invention, has been shown to couple to several signaling pathways including cyclic AMP production, mitogenesis and c-fos expression. Assays for these signals have been used to determine the function of such receptor ligands; however, the throughput of these assays is limited. The use of promiscuous and chimeric G proteins in conjunction with Fluorometric Imaging Plate Reader Systems (FLIPR) allows for the development of a single-platform functional assay that can be used to measure receptor activation. The present invention thus provides a general novel functional assay for certain receptors, including the D3 receptor, using a cell line that stably expresses both the particular receptor and the promiscuous G protein, for example, the HEK293-Gα15 cell line and a FLIPR³⁸⁴ measurement system to determine the functionality of such ligands. Unlike prior FLIPR methods, the assay method provided by the present invention relies on temperature-dependent agonist-induced activation of receptors, and affords a significantly enhanced signal over prior assay methods.

### Summary of the Invention

Accordingly, the present invention provides an assay method for determining activation by an agonist compound of a G-protein linked receptor, such as a neuroreceptor, said method being based on use of a Fluorometric Imaging Plate Reader, which comprises:
(a) generating a cell line having at least one suitable selection factor, selected from a drug resistance marker, selected from HEK293-G alpha15, said cell line stably expressing a promiscuous G protein selected from G alpha 15, and then co-expressing said G-linked receptor in said cell line, by transfecting cDNA coding for the selected G-linked receptor, into said cell line;
(b) growing the co-expressed cells in a suitable medium;
(c) plating said cells for approximately one day;
(d) loading the plated cells with an amount of a fluorescent dye suited to the purpose;
(e) incubating the dye-loaded cells at a temperature from about room temperature to about 37°C for about one hour;
(f) washing the plate to remove excess dye with a suitable buffer and replacing the volume of buffer removed with a similar volume of fresh buffer;
(g) incubating at from about 30°C to about 37°C ;
(h) adding an agonist compound under constant temperature conditions from about 30°C to about 37°C; and
(i) measuring fluorescence emission under constant temperature conditions from about 30°C to about 37°C in a Fluorometric Imaging Plate Reader so as to thereby determine the level of activation of the selected receptor by the agonist compound.

In one embodiment of the invention, the G-linked receptor is a dopamine or histamine receptor. In other embodiments, the G-linked receptor is selected from the group consisting of D2, D3, Alpha 1A, Alpha 2A, M1, H1, 5HT1A, and 5HT2A receptors. In another particular embodiment, said G-linked receptor is a dopamine D3 receptor.

In another embodiment of the invention, the selection factor selected from a drug resistance marker is a puromycin-resistance marker. In yet another embodiment of the invention, the selection factor selected from a drug resistance marker is a blastocidin-resistance marker.

A preferred fluorescent dye used in practising the method of the invention is Fluo-3™ or Fluo-4™. Preferably, the plated cells have a density of between about 12,000 and about 30,000 cells/square cm.

In another embodiment of the invention, incubating step (g) occurs for from about 15 minutes to about 60 minutes. Preferably, said incubating step (g) occurs for about 30 minutes.

### Brief Description of Drawings

Figure 1 shows the effect of temperature on agonist-dependent activation of dopamine D3 receptors.

Figure 2 shows the antagonism of dopamine-dependent intracellular calcium release by GR 218231, a D3-specific antagonist, at 37°C and 25°C (inset). Dopamine-induced Ca²⁺ release from intracellular stores was monitored by FLIPR.

Figure 3 shows agonist stimulation of D3 receptor-mediated G protein activation as measured by [³⁵S]-GTPγS binding assay. The experiment was performed at 25°C, 30°C, and 37°C as indicated. The receptor densities (Bₘₐₓ) at each temperature are presented.

Figure 4 shows the effect of temperature on agonist-dependent activation of histamine H1, 5HT1A, 5HT2A, dopamine D2, α-adrenergic 1A, α-adrenergic 2A and muscarinic M1 receptors as determined by FLIPR. Results are shown for measurements at 37°C and 25°C.

### Detailed Description of the Invention

As illustrated in the accompanying Figures, the present invention provides an assay method which uses a cell line that stably expresses both the receptor and the promiscuous G protein Gα15, or alternatively, uses the G protein subunits present in the cell. Agonist-induced intracellular Ca²⁺ release was monitored by use of a Fluorometric Imaging Plate Reader. In contrast to prior FLIPR-based assay methods where the assay is performed at 25°C, the magnitude of the agonist-induced response was dramatically enhanced by performing the assay at an elevated temperature, preferably at 37°C. While the EC50's of agonist activation determined by the FLIPR assay are higher than that determined by other functional receptor assays, functional Kᵢ's of inhibition by antagonists are similar. In the [³⁵S]GTPγS binding assay, another functional assay, agonist-induced activation of the receptor was also enhanced at elevated temperatures. Elevated temperature does not affect Bₘₐₓ of the receptors to which the present method applies, nor does it affect intracellular Ca²⁺ release induced by ionophore and endogenous purinergic receptor in the receptor-expressing cells.

Pharmacologically useful compounds which activate particular receptors may be discovered using the assay method of the present invention. Among the pharmacological uses of compounds selected by using the present assay method are, for example, the amelioration of the symptoms of anxiety, depression and other psychiatric conditions in a human subject, which would be identified by the ability of such compounds to activate dopamine receptors.

The present invention is illustrated, but not limited, by the following example.

### EXAMPLE

### D3 Protocol for HEK cells using FLIPR³⁸⁴

### Description of the cell line

The HEK293-G alpha15.D3 cell line stably expresses G alpha 15 which was generated by transfection of D3 cDNA into a HEK293 cell line expressing a G-alpha. The D3 receptor expression is maintained in the presence of selection factors such as puromycin and blastocidin. This cell line attaches poorly to typical tissue culture treated flasks. For a strongly adherent phenotype, the cells are grown on Matrigel (Becton Dickinson, diluted 1:200 with serum-free DMEM)-coated flasks.

Cell loading is carried out as follows:
(a) Plate 20,000 cells in 50 ul/well in a 384 well plate, or 60,000 cells per well in a 96 well plate, coated with poly D lysine. Return the plate to a 37°C incubator.
(b) Remove the growth media 16-24 hrs later and then replace the growth media with serum-free media in the presence of the calcium-sensitive fluorescent dye, fluo-4 (4 µM) and the active transport inhibitor probenecid (2.5 mM).
(c) Incubate the plate for one hour at 37°C.
(d) Aspirate the media and wash the plate with buffer (3 times, with HEPES-buffered saline containing probenecid; 2.5 mM) to remove excess dye.
(e) Incubate the plate for 15-45 minutes at 37°C.
(f) Preheat the plate to be loaded with a drug for 15 minutes in the 37°C incubator.
(g) Set up the assay in the FLIPR and monitor fluorescence levels continuously over a 90 sec period.

Agonist/antagonist additions (15 µl volume) were made simultaneously to all 96 or 384 wells after 20 sec of baseline recording. Antagonist pre-treatment times were 15 min.

### METHODS

### FLIPR Assay

The HEK293-Gα15 cell line stably expressing the D3 receptor was used to develop Ca²⁺-based assay using the FLIPR-based method. Cells were plated in a 96 or 384 well poly-D lysine-coated FLIPR plates for 16-24 hours before the assay. Cells were loaded with cell dye-media solution (media -11 ml, dye-4µM Fluo-4 AM, Pluronic® acid 22 ul, probenecid - 110ul of 260 mM solution) for 1 hour at 37°C. The plates were washed in the Skatron Embla™ plate washer with assay buffer (NaCl-0.145M, Glucose- 0.01 M, KCl-0.005M, MgSO₄-0.001M, HEPES-0.01M and CaCl₂-0.002M) and stabilized for 30 minutes at 25°C and 37°C, respectively, before starting the experiment. Antagonists were added 15 minutes prior to the agonist addition. The fluorescence intensities were measured using the excitation and emission wavelengths of 488 nm and 520 nm respectively. Data are taken as the average ± S.D. of four replicates.

### GTPγS Binding Assay

CHO cells expressing human D3 receptor were cultured in T175 flasks with medium containing DMEM and 10% fetal bovine serum. Cells were detached from the flask with 20 mM Hepes/10 mM EDTA and disrupted with a 22 1/2 gauge needle. After centrifuging at 40,000 x g, the membranes were resuspended in 20 mM Hepes/0.1 mM EDTA and centrifuged again. Membranes were resuspended in assay buffer (20 mM Hepes, 100 mM NaCI, 10 mM MgCl₂) and incubated with 1 µM GDP on ice for 10 min. The test compounds were assayed as follows: membranes and the test compounds were pre-incubated for 20 min at 25°C, 30°C and 37°C followed by 15 minutes incubation on ice. 10 µM GTPyS was added in some wells to define non-specific binding. To initiate the reaction, [³⁵S]-GTPγS was added at a final concentration of 0.1 nM. The assay plate was incubated for 30 minutes at 25°C, 30°C and 37°C. Wheat-germ agglutinin (WGA) SPA beads were then added to the assay (1 mg/well) and the assay plate shaken on a platform shaker for 30 minutes at room temperature. The plate was spun in a tabletop centrifuge for 5 minutes and counted on a Wallac Microbeta counter. Data were taken as the average ± S.D. of four replicates.

### ASSAY BUFFERS FOR D3 FLIPR ASSAY

Hepes Saline buffer is used for making compound dilutions and plate washings, and contains:

| "Buffer, M" | |
|---|---|
| NaCl | 0.145 |
| Glucose | 0.01 |
| KCI MgSO4 0.001 | 0.005 |
| HEPES 0.01 Ca(anhyd) | 0.002 |

| Probenecid Solution (260 mM) | |
|---|---|
| Probenecid | 0.74g |
| 5N NaOH | 1 ml |
| Hepes saline buffer | 9 ml |

"Fluo-3 or Fluo-4, AM Dye" is prepared by dissolving a 50 ug vial in 22ul DMSO.

| Cell media solution | The cells are incubated with this solution for dye loading |
|---|---|
| Serum free DMEM | 11 mls |
| Dye solution | 0.022 ml |
| 20% Pluronic® acid | 0.022 ml |
| Probenecid solution | 0.110 ml |

### Radioligand Binding Assay

CHO cells expressing D3 receptor were homogenized using a Polytron in 20 ml of assay buffer: 50 mM Tris, 120 mM NaCl, 5 mM KCI, 2mM CaCl₂, 5mM MgCl₂, pH 7.4. The homogenate was centrifuged at 20,000 RPM, 4°C for 10 min twice. The pellet was resuspended in assay buffer at a concentration of 5.0 mg/ml. Scatchard analysis was setup with varying concentrations of [³H] 7-OH-DPAT in a final volume of 250 ul. Plates were incubated for 60 min at 25°C, 30 min at 30°C and 15 min at 37°C. The reaction was stopped by rapid filtration through GF/B filters (previously soaked in 0.5% PEI for 2 hours and dried) with ice cold 50 mM Tris buffer at pH 7.4 in the Skatron harvester. Filters were counted in the Beta counter using Betaplate Scint.

### RESULTS

### D3 agonist-stimulated intracellular Ca²⁺ response is temperature-sensitive: FLIPR assay

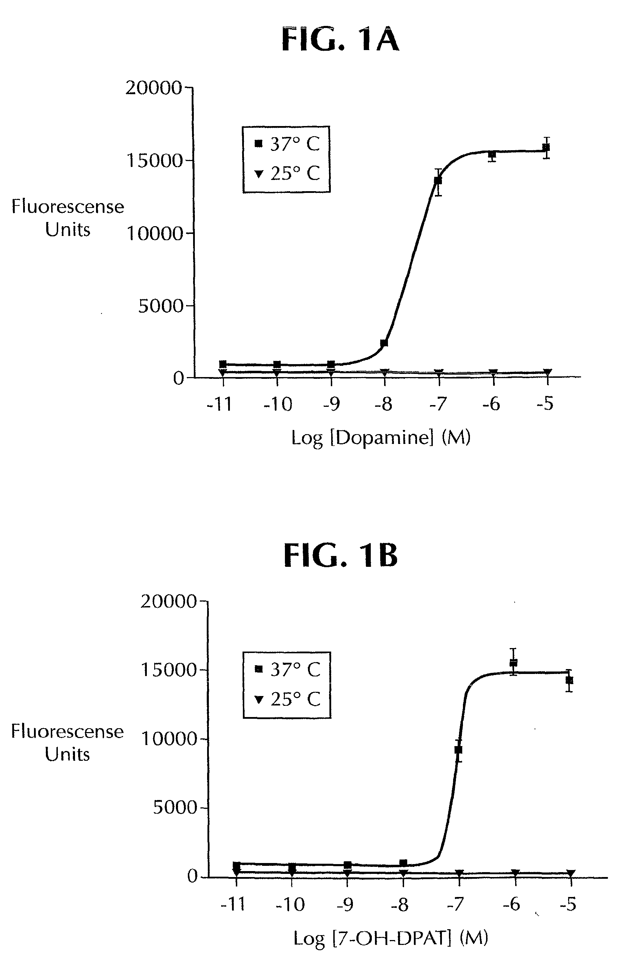

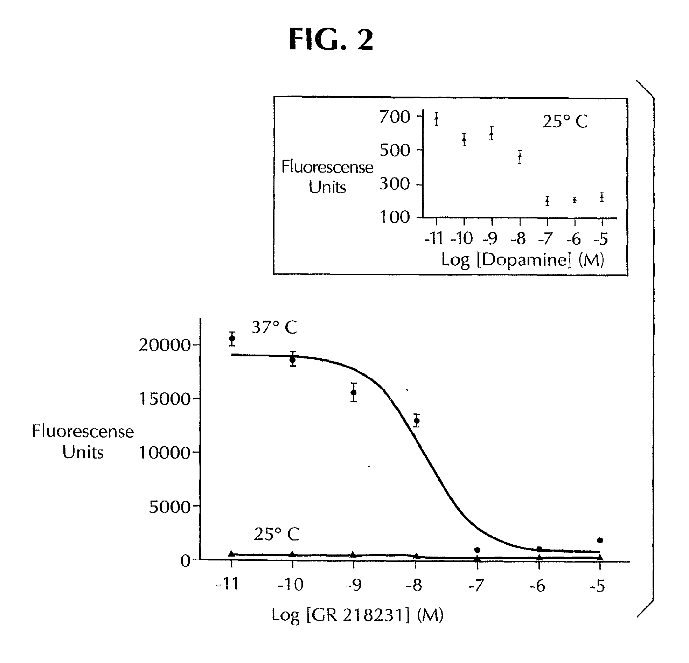

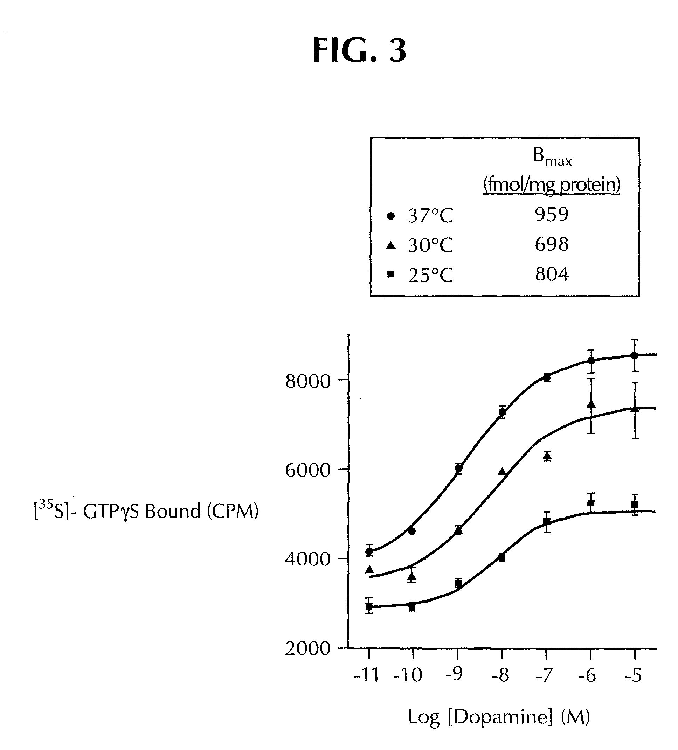

Fig. 1 shows agonist-stimulated intracellular Ca²⁺ release from the HEK293-Gα15 cell line stably expressing the D3 receptor. The assay was performed in the presence of indicated concentrations of dopamine and 7-OH-DPAT and the agonist induced Ca²⁺ release was determined by FLIPR.

There is a dramatic temperature-dependent increase of fluorescence signal at 37°C as compared to 25°C. The agonist-mediated response is D3 receptor-specific since 7-OH-DPAT is a selective D3 agonist. The assay is rapid, reproducible, and sensitive, and thus useful for high throughput screening to detect D3 agonists.

Fig. 2 shows the effect of GR 218231, a D3 specific antagonist, on the dopamine-induced Ca²⁺ release as monitored by FLIPR. HEK293-Gα15 cell line stably expressing the D3 receptor were pre-incubated with indicated concentrations of GR 218231 for 15 minutes prior to the addition of 100 nM dopamine at 37°C and 25°C (inset). The dopamine-induced FLIPR response is D3-mediated. Similar functional IC₅₀ values were observed at both temperatures, suggesting that elevated temperature do not affect antagonist binding. The functional Kᵢ of GR 218231 was similar to published values, suggesting that the assay can be used to determine functional Kᵢ of D3 antagonists.

### G-protein activation bv D3 receptor agonist: [³⁵S]-GTPγS binding assay

Fig. 3 shows that the temperature-dependent effect of agonist-mediated activation of D3 receptors is also observed in [³⁵S]-GTPγS assay, another D3 functional assay. The amount of [³⁵S]-GTPγS bound to the G-protein is a measure of agonist activation. Bₘₐₓ of D3 binding sites at each temperature is tabulated at the inset. Similar to that observed in FLIPR, the agonist-induced signal was higher at elevated temperatures, although the effect is less pronounced. Elevated temperatures do not appear to affect the Bₘₐₓ of D3 in the membrane preparations.

### Summary of agonist EC₅₀ and antagonist functional Kᵢ

Table 1 shows a comparison of agonist and antagonist values as determined by FLIPR and [³⁵S]-GTPγS assays at various temperatures. There is a 10-fold increase in the EC₅₀ of dopamine in the FLIPR versus the GTPγS assay, which may be a function of two different cell lines used. The CHO-D3 cell line used for the GTPγS assay utilizes the endogenous G proteins whereas the HEK293 cell line used for the FLIPR assay utilizes Gα15. The functional Kᵢ values of antagonists determined by the two assays are similar, suggesting that the FLIPR assay can be used to determine the functional Kᵢ of D3 antagonists.

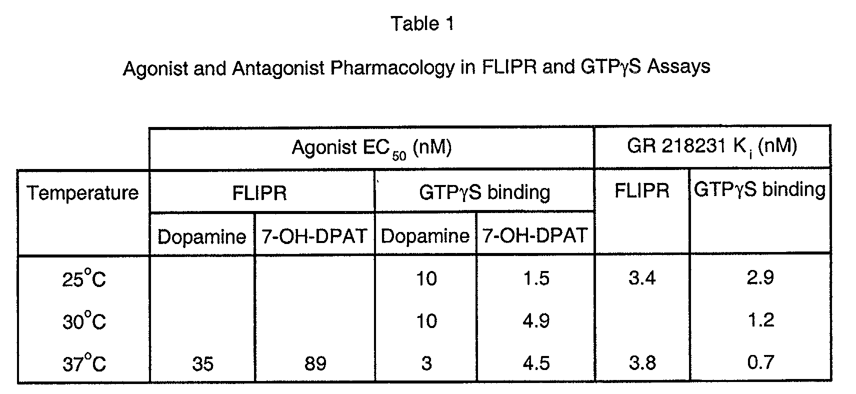

**Temperature-dependent intracellular calcium release is observed in many GPCRs.**

Table 2 shows intracellular Ca²⁺release measured by the FLIPR assay in the D3-Gα15 cells. Concentrations that are at or above the EC₉₀ were used: dopamine (100 nM), ATP (12.5 mM), ionomycin (10 mM). The elevation of FLIPR signal (in percentage) in 5 min and 15 min pre-incubation at 37°C is shown.

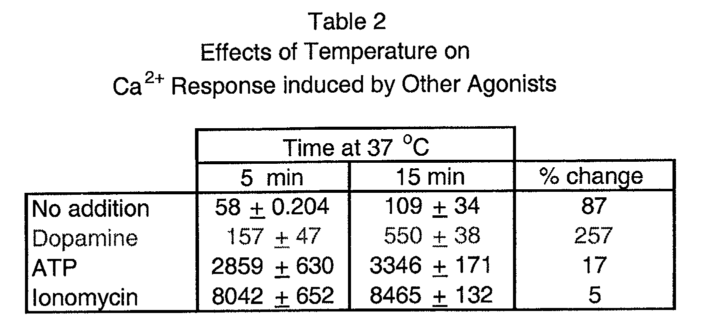

While there is an elevation of agonist response by D3 agonist at elevated temperature, the response induced by ATP (via endogenous purinergic receptors) and ionomycin (ionophore) was not affected by prolonged preincubation at 37°C. The above results indicate that temperature-dependent activation of G protein-coupled receptors (GPCRs) is observed for D3 but not for purinergic receptor, and intracellular release of Ca²⁺ is not affected by elevated temperature. Without being bound by a theory, the enhanced agonist response may be related to the coupling of D3 receptors to G proteins and extended as a general mechanism of activation of many GPCRs.

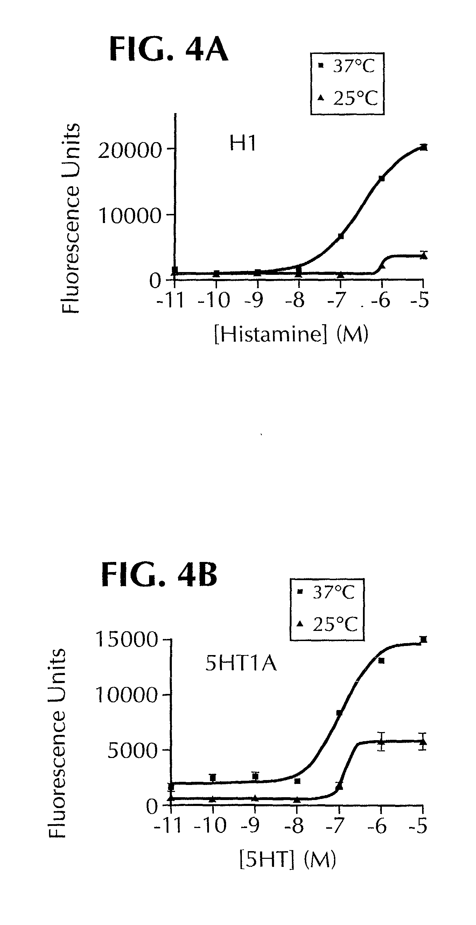

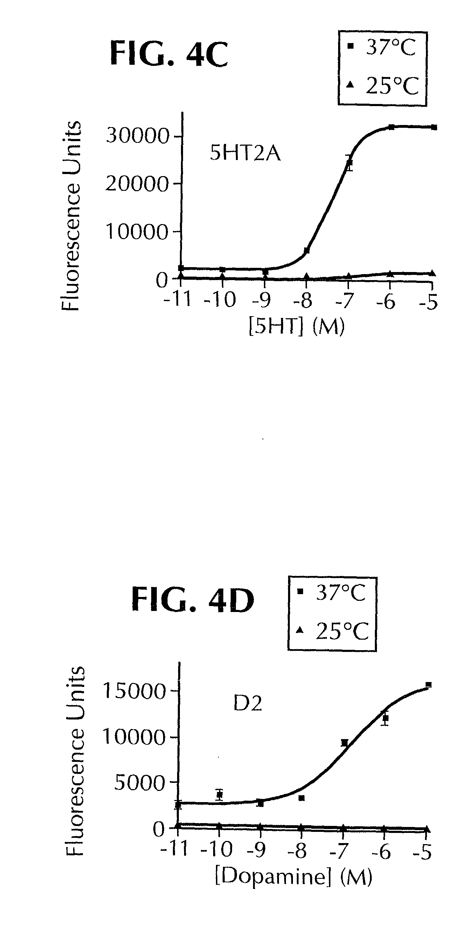

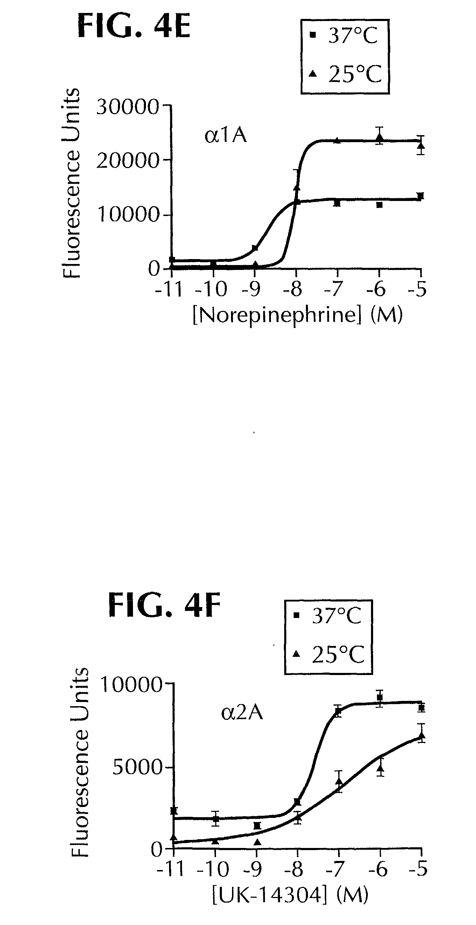

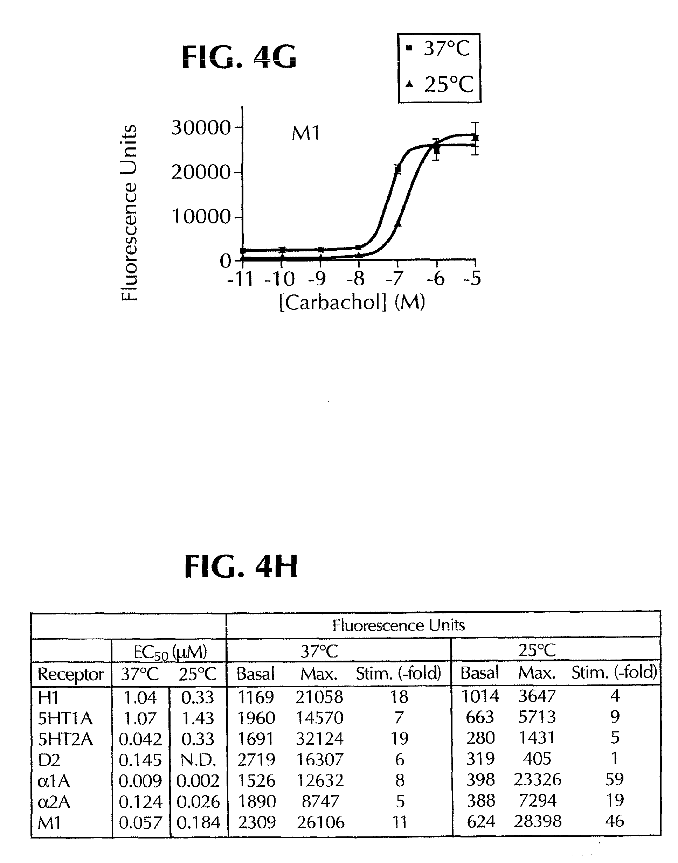

Fig. 4 shows the agonist-dependent activation in FLIPR for a variety of GPCRs: α-adrenergic 1A, α-adrenergic 2A, histamine H1, 5HT1A, 5HT2A, dopamine D2 and muscarinic M1. Within this subset of GPCRs, the magnitude of agonist-dependent response was increased at elevated temperature for a majority (>70%) of these GPCRs. All of these receptors activate the release of intracellular Ca²⁺ via G protein subunits present in the cell (G_{q} or Gᵢ). These data provide evidence that the above-identified phenomenon is observed for many GPCRs and that assay performance at elevated temperature will enable better detection of agonist-dependent response.

The present invention therefore provides a FLIPR-based, novel, rapid and reproducible functional assay for the various receptors. Functional assays measuring intracellular calcium release (FLIPR) and GPCR activation (GTPγS binding) demonstrate increased signal at 37°C as compared to 25°C. Functional Kᵢ values of antagonists generated by both functional assays are not affected by elevated temperature.

## Claims

1. An assay method for determining activation by an agonist of a G-protein linked receptor, said method being based on use of a Fluorometric Imaging Plate Reader, which comprises:
(a) generating a cell line having at least one suitable selection factor, selected from a drug resistance marker, selected from HEK293-G alpha15, said cell line stably expressing a promiscuous G protein selected from G alpha 15, and then co-expressing a said G-linked receptor in said cell line, by transfecting cDNA coding for the selected G-linked receptor, into said cell line;
(b) growing the co-expressed cells in a suitable medium;
(c) plating said cells for approximately one day;
(d) loading the plated cells with an amount of a fluorescent dye suited to the purpose;
(e) incubating the dye-loaded cells at a temperature from about room temperature to about 37°C for a suitable period;
(f) washing the plate to remove excess dye with a suitable buffer and replacing the volume of buffer removed with a similar volume of fresh buffer;
(g) incubating at from about 30°C to about 37°C;
(h) adding an agonist under constant temperature conditions from about 30°C to about 37°C; and
(i) measuring fluorescence emission under constant temperature conditions from about 30°C to about 37°C in a Fluorometric Imaging Plate Reader so as to thereby determine the level of activation of the selected receptor by the agonist compound.

2. The method of claim 1 wherein said G-linked receptor is a dopamine or histamine receptor

3. The method of claim 1 wherein said G-linked receptor is selected from the group consisting of D2, D3, Alpha 1A, Alpha 2A, M1, H1, 5HT1A, and 5HT2A receptors.

4. The method of claim 1 wherein said G-linked receptor is a dopamine D3 receptor.

5. The method of any one of claims 1 to 4 wherein said selection factor selected from a drug resistance marker is a puromycin-resistance marker.

6. The method of any one of claims 1 to 4 wherein said selection factor selected from a drug resistance marker is a blastocidin-resistance marker.

7. The method of any preceding claim wherein said fluorescent dye is Fluo-3™ or Fluo-4™.

8. The method of any preceding claim wherein the plated cells have a density of between about 12,000 and about 30,000 cells/square cm.

9. The method of any preceding claim wherein said incubating step (e) occurs for about one hour.

10. The method of any preceding claim wherein said incubating step (g) occurs for from about 15 minutes to about 60 minutes.

11. The method of any preceding claim wherein said incubating step (g) occurs for about 30 minutes.
